(19) Europäisches Patentamt
European Patent Office
Office européen des brevets

(11) **EP 3 092 987 A1**

(12) **EUROPÄISCHE PATENTANMELDUNG**

(43) Veröffentlichungstag:
16.11.2016 Patentblatt 2016/46

(51) Int Cl.:
*A61F 13/00* (2006.01)    *B01D 61/02* (2006.01)
*A61K 35/16* (2006.01)

(21) Anmeldenummer: **15167089.0**

(22) Anmeldetag: **11.05.2015**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA**

(71) Anmelder: **3M Innovative Properties Company St. Paul, MN 55133-3427 (US)**

(72) Erfinder: **Bonn, Florian 51379 Leverkusen (DE)**

(74) Vertreter: **CPW GmbH Kasinostraße 19-21 42103 Wuppertal (DE)**

(54) **SYSTEM ZUR WUNDBEHANDLUNG MIT SERUM**

(57) System zur Wundbehandlung mit Serum mit einer Vorrichtung zur Gewinnung von Serum aus Vollblut, welche umfasst:
- einen Blutbeutel zur Aufnahme und zur Koagulation von Vollblut, der einen Auslass zur Ableitung der das Serum enthaltenden Flüssigkeit aus dem Blutbeutel aufweist und im Bereich des Auslasses eine Sperre zum Rückhalt des Koagulums,
- einen mit dem Blutbeutel in Fluidverbindung stehenden Filtermodul mit einem Gehäuse und einem Innenraum, in dem eine semipermeable Membran angeordnet ist, die den Innenraum in einen Retentatraum und einen Permeatraum unterteilt und eine Auftrennung der aus dem Blutbeutel abgeleiteten Flüssigkeit in ein das Serum umfassendes Permeat und in ein Retentat, in dem eventuell in der Flüssigkeit enthaltene partikulärer Bestandteile verbleiben, ermöglicht.
Das System zur Wundbehandlung umfasst des Weiteren ein Wundversorgungssystem mit einem Kapillarmembransystem zum Ausbringen des Serums auf eine Wunde. Das Kapillarmembransystem ist mit mindestens einer Versorgungsleitung verbunden, die ihrerseits mit dem Permeatauslass des Filtermoduls verbunden ist.

Fig. 1

EP 3 092 987 A1

**Beschreibung**

Die Erfindung betrifft ein Wundversorgungssystem zum Einbringen in eine Wunde bzw. zum Aufbringen auf eine Hautwunde und unter einen Wundverband, umfassend ein flächenförmig ausgebildetes Kapillarmembransystem, wobei das flächenförmig ausgebildete Kapillarmembransystem mit mindestens einer Versorgungsleitung verbunden ist, so dass durch die Versorgungsleitung und das Kapillarmembransystem Flüssigkeiten, Medien, Gase und/oder andere Stoffe durchleitbar sind.

Die moderne Wundversorgung verfolgt das Ziel, bei der Wundversorgung ein feuchtes Wundmileu zu schaffen, das bei der Heilung ablaufende Prozesse fördert. Je nach Heilungsphase müssen moderne, aktive Wundauflagen deshalb in der Lage sein, die Wunde feucht zu halten, für einen verbesserten Flüssigkeits-/Stoffaustausch, für das Einbringen von Faktoren/Medikamenten und/oder für eine verbesserte Flüssigkeits-/Sekrets- und/oder Stoffentfernung in der Wunde bzw. in die Wunde zu sorgen. Anwendungen schließen die Nutzung solcher Wundauflagesysteme in einer Weichteilwunde, in einer abdominellen Wunde und auf einer Hautwunde ein.

Ein Verfahren und eine Vorrichtung zur Entfernung von Sekret bzw. Exsudat aus Wunden ist kommerziell bekannt als V.A.C.® Therapy System (Fa. KCI, USA). Bei diesem System wird für ein alternierendes Einbringen von Flüssigkeit in die Wunde und ein nachfolgendes also auch alternierendes und damit nicht kontinuierliches Ausführen von Flüssigkeit aus der Wunde gesorgt. Ein in die Wunde eingebrachtes Schaumstoffmaterial, das bei Unterdruck Kräfte auf die Wunde ausübt, soll bei diesem System die Wundheilung begünstigen.

In der DE 10 2006 042 732 wird ein Kapillarmembransystem zur Wundbehandlung beschrieben, bei dem die Wunde über eine Hohlfasermembrananordnung aus bis zu 1000 Hohlfasern mit mindestens einer gemeinsamen Zu- und mindestens einer gemeinsamen Ableitung im Sinne der Durchströmung eines Kapillarbettes perfundiert und versorgt werden und eine Antibiotika- sowie Wachstumsfaktorenperfusion ermöglicht werden soll. Dabei soll eine gleichmäßige Stoffverteilung unter kontinuierlicher Perfusion auch unter Erzeugung eines moderaten Unterdruckes ermöglicht werden. Die DE 10 2006 042 732 führt aus, dass für eine optimale Ver- und Entsorgung weitere Kapillarmembransysteme von Vorteil sind.

Wenngleich sich bereits mit den in der DE 10 2006 042 732 beschriebenen Kapillarmembransystemen Fortschritte bei der Wundbehandlung erzielen lassen, besteht Bedarf an einfachen und effizienten Wundversorgungssystemen, mit denen zum einen die bei der Wundbehandlung notwendigen Vorgänge, wie Spülen und Desinfizieren, ohne Entfernen des Verbandes durchgeführt werden können, die erforderlichenfalls eine Ernährung, Elektrolytaustausch und/oder Detoxifikation oder auch eine Wachstumsfaktorenversorgung oder eine Versorgung mit Antibiotika ermöglichen und die eine einfache und sichere Handhabung erlauben.

In zunehmenden Maße wird auch an Wundbehandlungen gearbeitet, bei denen Eigen- oder auch Fremdserum der Wunde zugeführt wird, um den Heilungsprozess zu beschleunigen. Die Wundbehandlung mit Serum kann sowohl kontinuierlich, als auch diskontinuierlich erfolgen. Die ideale Applikation ist wundabhänging und muss individuell für den zu behandelnden Patienten festgelegt werden.

Daher besteht der Wunsch nach einem Wundversorgungssystem, das die gezielte Einbringung von Blutserum in die zu behandelnde Wunde ermöglicht. Es ist daher Aufgabe der vorliegenden Erfindung, ein derartiges System zur Wundbehandlung zur Verfügung zu stellen.

Die Aufgabe wird durch ein System zur Wundbehandlung mit Serum gelöst, welches eine Vorrichtung zur Gewinnung von Serum aus Vollblut umfasst, wobei die Vorrichtung zur Gewinnung von Serum umfasst:

-   einen Blutbeutel zur Aufnahme von Vollblut und zur Koagulation des Vollbluts in einen die Zellbestandteile des Bluts enthaltenden Feststoffanteil und in eine Serum enthaltene Flüssigkeit, wobei der Blutbeutel einen Auslass zur Ableitung der Flüssigkeit aus dem Blutbeutel aufweist und im Bereich des Auslasses eine Sperre, welche zum Rückhalt zumindest eines Großteils des Feststoffanteils geeignet ist,
-   einen mit dem Blutbeutel über den Auslass des Blutbeutels in Fluidverbindung stehenden Filtermodul,

    -   wobei der Filtermodul ein Gehäuse mit einem Innenraum und einer den Innenraum begrenzenden Innenwand aufweist, in dem eine semipermeable Membran angeordnet ist, die den Innenraum in einen Retentatraum und einen Permeatraum unterteilt,
    -   wobei die semipermeable Membran eine Auftrennung der aus dem Blutbeutel abgeleiteten Flüssigkeit in ein Permeat, welches das Serum umfasst, und in ein Retentat, in dem eventuell in der Flüssigkeit enthaltene partikulärer Bestandteile verbleiben, ermöglicht und
    -   wobei der Filtermodul eine Einlasseinrichtung zum Einleiten der aus dem Blutbeutel abgeleiteten Flüssigkeit in den Retentatraum, einen Retentatauslass zum Ableiten von Retentat aus dem Retentatraum und einen Permeatauslass zum Ableiten des Permeats aus dem Permeatraum aufweist, und

wobei das System zur Wundbehandlung des Weiteren ein Wundversorgungssystem zum Ausbringen des Permeats auf eine Wunde umfasst, welches ein erstes flächenförmig ausgebildetes Kapillarmembransystem aufweist, wobei das erste Kapillarmembransystem mit mindestens einer Versorgungsleitung verbunden ist, so dass durch die Versorgungsleitung und das erste Kapillarmembransystem Flüssigkeiten hindurchdurchleitbar und der Wunde zuführbar sind, und wobei der Permeatauslass über eine die mindestens eine Versorgungsleitung des ersten Kapillarmembransystems umfassende Permeatleitung mit dem ersten Kapillarmembransystem verbunden ist.

Mittels eines solchen Systems zur Wundbehandlung ist eine direkte Versorgung einer Wunde mit Serum möglich, wobei gleichzeitig eine nahezu homogene Verteilung des Serums in der Wunde möglich ist. Dabei erlaubt die Konfiguration des Wundversorgungssystems gleichzeitig eine sichere und einfache Handhabung. In der Anwendung kann dazu das Wundversorgungssystem in die Wunde eingelegt und z.B. mittels einer semiokklusiven transparenten Folie abgedeckt werden, um die Wunde vor dem Austrocknen oder vor Infektionen zu schützen.

Über die mindestens eine Versorgungsleitung des ersten Kapillarmembransystems steht das erste Kapillarmembransystem mit der Vorrichtung zur Gewinnung von Serum in Fluidverbindung. Das im Filtermodul erzeugte Blutserum kann damit über das erste Kapillarmembransystem in die Wunde eingebracht werden.

Als Vorrichtung zur Gewinnung von Serum aus Blut können im Prinzip Vorrichtungen eingesetzt werden, wie sie insbesondere zur Plasmaseparation, d.h. zur Gewinnung von Blutplasma und Erythrozytenkonzentraten unter Einwirkung von Schwerkraft eingesetzt werden. Derartige Vorrichtungen werden beispielsweise in der DE-U-29 516 471, der EP-A-0 266 683, der EP-A-1 089 778, der EP-A-1 309 363, der US-A-4 639 243 oder der WO 2011/157822 beschrieben.

Diese Vorrichtungen umfassen, wie auch die vorliegende Vorrichtung zur Gewinnung von Serum aus Vollblut, einen Blutbeutel zur Aufnahme einer ausreichenden Menge an Vollblut, einen mit dem Blutbeutel verbundenen Filtermodul, in dem im Falle der Plasmaseparation eine Auftrennung des Bluts in Blutplasma und Erythrozytenkonzentrat vorgenommen wird, wobei bei dieser Anwendung eine getrennte Ableitung dieser Komponenten in entsprechende Aufnahmebeutel erfolgt.

Wie bei der zuvor erwähnten Plasmaseparation unter der Einwirkung der Schwerkraft, kann auch beim vorliegenden System zur Wundbehandlung die Gewinnung des Serum und die Zuführung des Serums in den Wundbereich unter der Einwirkung der Schwerkraft, d.h. ohne zusätzlich Pumpen oder durch Beaufschlagung mit Druck o.ä., erfolgen. Hierzu können in der Anwendung Blutbeutel und Filtermodul über eine geeignete Haltevorrichtung in einem definierten senkrechtem Abstand zueinander und über der Wunde angeordnet werden, so dass das Ausfließen aus dem Blutbeutel in den Filtermodul und im weiteren aus dem Filtermodul und über das erste Kapillarmembransystem in den Wundbereich unter dem Einfluss der Schwerkraft erfolgt.

Für die vorliegende Vorrichtung zur Gewinnung von Serum ist in der Anwendung der Blutbeutel mit einer vorgegebenen Menge an Vollblut gefüllt. Das im Blutbeutel befindliche Blut wird dann einer Koagulation oder Blutgerinnung unterworfen. Die Koagulation ist ein komplexer Vorgang, der wie eine Kettenreaktion unter Beteiligung verschiedenen Gerinnungsfaktoren (wie Thrombozyten, Fibrinogen, Calcium und Vitamin K) abläuft. Die Koagulation des Blutes wird durch die Entnahme des Blutes und das Fehlen von Antikoagulationsmitteln wie Citrat oder Heparin im Blutbeutel ausgelöst.

Infolge der Blutgerinnung trennt sich das Blut in einen Feststoffanteil (Koagulum), der die zellulären Bestandteile wie z.B. rote Blutkörperchen, Blutplättchen und weiße Blutkörperchen enthält und eine flüssige Phase, die bis auf die bei der Gerinnung verbrauchten Gerinnungsfaktoren alle natürlicherweise in der Blutflüssigkeit gelösten Stoffe enthält. Dabei liegt der Feststoffanteil als gallertartige Masse vor.

Der Blutbeutel der vorliegenden Vorrichtung zur Gewinnung von Serum umfasst im Bereich seines Auslasses eine Sperre, welche zum Rückhalt zumindest eines Großteils des Feststoffanteils, d.h. des in Gestalt einer gallertartigen Masse vorliegenden Koagulums geeignet ist. Hierzu kann die Sperre eine Verengung im Bereich des Auslasses sein, die den Durchtritt des Flüssigkeitsanteils erlaubt. Die Sperre kann auch eine Klemme sein, die im Bereich des Auslasses einen genügend großen Spalt für den Durchtritt der Flüssigkeit aus dem Blutbeutel aufweist.

Der Auslass des Blutbeutels steht mit dem Filtermodul der Vorrichtung zur Gewinnung von Blutserum in Fluidverbindung, beispielsweise über einen geeigneten Schlauchabschnitt, wobei die Verbindung zwischen Blutbeutel und Filtermodul über den Schlauchabschnitt vorzugsweise lösbar ausgeführt ist, beispielsweise durch geeignete Male-/Female-Konnektoren etwa in Gestalt von Luer-Lock-Konnektoren. Alternativ kann die Verbindung zwischen Blutbeutel und Filtermodul, d.h. letztlich zwischen Blutbeutel und Schlauchabschnitt, zwischen Schlauchabschnitt und Filtermodul oder zwischen Teilabschnitten des Schlauchabschnitts auch als sterile Schweißverbindung ausgeführt sein, wie sie mittels eines Sterilkonnektors (z.B. TSCD® II Sterile Tubing Welder, Fa. Terumo) hergestellt werden kann. Der Filtermodul seinerseits bzw. der Permeatauslass des Filtermoduls steht über die Permeatleitung, die mindestens eine Versorgungsleitung des ersten Kapillarmembransystems umfasst, in Fluidverbindung mit dem ersten Kapillarmembransystem. Vorzugsweise ist auch die Verbindung zwischen Permeatauslass des Filtermoduls und erstem Kapillarmembransystem lösbar, z.B. durch geeignete Male-/Female-Konnektoren, die vorzugsweise Luer-Lock-Konnektoren sein können oder kann als sterile Schweißverbindung ausgeführt sein, wie sie mittels der zuvor genannten Sterilkonnektoren hergestellt werden kann. In der Permeatleitung kann ein Brechventil angeordnet sein, so dass eine gezielte Zugabe des Serums zu einem definierten Zeitpunkt erfolgen kann.

Die im Filtermodul bzw. im Innenraum des Gehäuses des Filtermoduls angeordnete semipermeable Membran kann in Gestalt einer Flachmembran oder einer Hohlfasermembran ausgeführt sein. Bevorzugt ist die semipermeable Membran ein Bündel von Hohlfasermembranen. In diesem Fall wird der Retentatraum durch die Lumina der Hohlfasermembranen und der Permeatraum durch den die Hohlfasermembranen umgebenden und durch die Innenwand des Gehäuses begrenzten Außenraum ausgebildet. Dabei ist in einer besonders bevorzugten Ausführungsform das im Filtermodul angeordnete Bündel von Hohlfasermembranen U-förmig ausgebildet. In diesem Fall hat das Gehäuse des Filtermoduls einen Deckel und einen Boden und die Einlasseinrichtung zum Einleiten der aus dem Blutbeutel abgeleiteten Flüssigkeit in den Retentatraum sowie der Retentatauslass zum Ableiten von Retentat aus dem Retentatraum sind deckelseitig angeordnet. Der Permeatauslass zum Ableiten des Permeats, d.h. des Serum, aus dem Permeatraum ist bei diesem Filtermodul bodenseitig angeordnet.

Um einen genügend hohen Permeatfluss durch die semipermeable Membran des Filtermoduls insbesondere bei der Gewinnung von Serum unter dem Einfluss der Schwerkraft generieren zu können, weist die semipermeable Membran des Filtermoduls vorzugsweise einen Transmembranfluss im Bereich von 10.000 bis 40.000 l/(m$^2$·h·bar) auf. In einer weiteren bevorzugten Ausführungsform weist die semipermeable Membran des Filtermoduls eine nominelle Pore von 0,2 $\mu$m auf. Dabei wird die nominelle Pore über das Rückhaltevermögen der Membran gegenüber spezifischen Mikroorganismen definiert. Membranen mit einer nominellen Pore von 0,2 $\mu$m werden generell als bakteriendicht bezeichnet, da sie Bakterien der Gattung Brevundimonas diminuta zurückhalten. Ebenso halten sie natürlich z.B. auch Bakterien der Gattung Serratia marcescens zurück, für die eine Membran mit nomineller Pore von 0,45 $\mu$m ausreichend wäre. Die Prüfungen bzw. die Ermittlung der nominellen Porengrößen sind beispielsweise in der HIMA-Vorschrift No. 3, Vol. 4, 1982 (Health Industry Manufacturers Association) beschrieben.

Wie ausgeführt steht der Permeatauslass des Filtermoduls der Vorrichtung zur Gewinnung von Serum über die Permeatleitung, die mindestens eine Versorgungsleitung des ersten Kapillarmembransystems umfasst, in Fluidverbindung mit dem ersten Kapillarmembransystem. Dabei kann in einer bevorzugten Ausführungsform In der Permeatleitung mindestens ein Aufnahmebehälter mit einem Einlass und einem Auslass zur Aufnahme des Permeats bzw. des aus dem Filtermoduls austretenden Serums angeordnet sein. Dabei ist der Einlass des Aufnahmebehälters mit dem Permeatauslass des Filtermoduls über einen dem Filtermodul zugewandten Teilabschnitt der Permeatleitung verbunden und der Auslass des Aufnahmebehälters über eine Ablaufleitung als Teil der Permeatleitung mit dem ersten Kapillarmembransystem.

In einer weiteren vorteilhaften Ausführungsform besteht der Aufnahmebehälter aus einer Mehrzahl von Teilaufnahmebehältern, die in Parallelschaltung zueinander angeordnet sind. Die Auslässe der Teilaufnahmebehälter sind jeweils mit einer Ablaufleitung verbunden, die über ein Verbindungselement letztlich mit der Versorgungsleitung des ersten Kapillarmembransystems verbunden sind. Dabei weisen die Ablaufleitungen vorzugsweise jeweils ein Regelorgan auf, mittels dessen der jeweilige Teilaufnahmebehälter zu- bzw. abgeschaltet oder mittels dessen der Durchfluss durch die Ablaufleitung auf definierte Werte eingestellt werden kann. Eine solche Ausführungsform ist z.B. insbesondere dann von Vorteil, wenn das im Filtermodul erzeugte Serum in Aliquots auf mehrere Teilaufnahmebehälter aufgeteilt werden soll, um anschließend bei der Wundbehandlung die Aliquots des Serums nacheinander zu bestimmten Zeitabschnitten zuzuführen.

In einer bevorzugten Ausführungsform umfasst das vorliegende System zur Wundbehandlung mit Serum neben dem ersten Kapillarmembransystem mindestens ein weiteres, flächenförmig ausgebildetes Kapillarmembransystem, über das der Wunde weitere Flüssigkeiten oder Wirksubstanzen oder Gase zugeführt werden können oder über das eine Entfernung von Flüssigkeiten aus dem Wundbereich vorgenommen werden kann. In einer besonders bevorzugten Ausführungsform umfasst das vorliegende System zur Wundbehandlung zweites flächenförmig ausgebildetes Kapillarmembransystem, welches mit mindestens einer Versorgungsleitung verbunden ist, so dass durch die Versorgungsleitung und das zweite Kapillarmembransystem Flüssigkeiten, Medien, Gase und/oder andere Stoffe durchleitbar sind, sowie einen Entnahmebehälter, welcher über einen Leitungsweg, der mindestens eine der Versorgungsleitungen des zweiten Kapillarmembransystems umfasst, mit dem zweiten Kapillarmembransystem lösbar verbunden ist und über den dem zweiten Kapillarmembransystem eine Spülflüssigkeit zuführbar ist. Ebenso kann das System zur Wundbehandlung ein Drainagesystem umfassen, welches mit einer Unterdruckeinheit koppelbar ist und über das Flüssigkeiten aus der zu behandelnden Wunde abführbar sind.

In der Anwendung können das erste und gegebenenfalls vorhandene weitere Kapillarmembransysteme in die Wunde eingelegt und, wie bereits ausgeführt, z.B. mittels einer semiokklusiven transparenten Folie abgedeckt werden, um die Wunde vor dem Austrocknen oder vor Infektionen zu schützen. Die Versorgungsleitungen der Kapillarmembransysteme werden dann unter der Folie aus dem Wundbereich herausgeführt und sind außerhalb des Wundbereichs mit dem Filtermodul der Vorrichtung zur Gewinnung von Serum bzw. mit jeweils zugeordneten Entnahmebehältern usw. verbunden. Das gegebenenfalls vorhandene Drainagesystem kann mit einer Unterdruckeinheit z.B. über einen geeigneten, gegen Unterdruck stabilen Schlauch in Verbindung stehen, der ebenfalls aus dem Wundbereich herausgeführt wird.

Hierbei kann das erste bzw. können die gegebenenfalls vorhandenen weiteren flächenförmig ausgebildeten Kapillarmembransysteme (im Folgenden als das mindestens eine Kapillarmembransystem bezeichnet) jeweils aus einer einzelnen Kapillarmembran bestehen, die mäanderförmig angeordnet ist. Bei dieser Ausführungsform ist mindestens eines der Enden der mäanderförmigen Kapillarmembran offen und mit einer Versorgungsleitung verbunden. Das mindestens eine Kapillarmembransystem kann jedoch auch mehrere mäanderförmig angeordnete Kapillarmembranen umfassen, die zusammen mit ihren Enden in eine gemeinsame Versorgungsleitung münden. Vorzugsweise umfasst das mindestens eine Kapillarmembransystem eine Mehrzahl von zueinander parallel angeordneten Kapillarmembranen.

Die zueinander parallel angeordneten Kapillarmembranen eines jeweiligen Kapillarmembransystems sind an mindestens einem ihrer Enden so an ihrem äußeren Umfang fluiddicht in der Wand einer Versorgungsleitung eingebettet, dass zwischen dem Lumen der Versorgungsleitung und dem Lumen der Kapillarmembranen eine Fluidverbindung besteht und durch die Versorgungsleitung und das mindestens eine Kapillarmembransystem Flüssigkeiten, Medien, Gase und/oder andere Stoffe durchleitbar sind. Die Einbettung kann beispielsweise mit einem härtbaren Silikonmaterial, mit einem Polyurethan- oder einem Epoxidharz erfolgen. Bevorzugt werden wegen ihrer besseren Flexibilität härtbare Silikonmaterialien eingesetzt. Im Falle, dass die Kapillarmembranen nur mit einem ihrer Enden in einer Versorgungsleitung eingebettet sind, ist das andere, gegenüberliegende Ende der Kapillarmembranen geschlossen, beispielsweise durch Verschweißen oder Verkleben. Die Kapillarmembranen können auch an ihren beiden Enden offen und mit diesen beiden Enden auf einer Seite der Anordnung in eine einzelne Versorgungsleitung eingebettet sein, wobei die Kapillarmembranen dann an ihrem freien Ende U-förmig ausgebildet sind und dadurch dort geschlossen sind. In diesen Fällen werden die Kapillarmembranen im Dead-end Modus betrieben.

Insbesondere bei breiteren Wundversorgungssystemen sind Ausführungsform der Kapillarmembransysteme mit parallel zueinander angeordneten Kapillarmembranen von Vorteil, bei dem die Kapillarmembranen an ihren beiden Enden offen und in jeweils eine Versorgungsleitung eingebettet sind, wobei sich die Versorgungsleitungen sich dann bevorzugt an gegenüberliegenden Seiten der des jeweiligen flächenförmig ausgebildeten Kapillarmembransystems befinden. Auch in diesem Fall ist die Einbettung so ausgeführt, dass die Kapillarmembranen an ihrem äußeren Umfang fluiddicht eingebettet sind und zwischen dem Lumen der jeweiligen Versorgungsleitung und dem Lumen der Kapillarmembranen eine Fluidverbindung besteht. Eine derartige Ausführungsform mit zwei Versorgungsleitungen erlaubt eine Versorgung und/oder Entsorgung über das mindestens eine Kapillarmembransystem im cross-flow Modus. Auch mit Blick auf eine gute Homogenität der Versorgung bzw. Entsorgung über der Fläche der Wunde kann insbesondere bei breiteren Matten bzw. Wundversorgungssystemen die Ausführung des mindestens einen Kapillarmembransystems mit zwei Versorgungsleitungen zweckmäßig sein. Eine Ausführungsform mit zwei Versorgungsleitungen ermöglicht jedoch auch eine gleichzeitige oder auch eine alternierende Versorgung einer Wunde mit unterschiedlichen Medien über dasselbe Kapillarmembransystem.

Der Durchmesser der Versorgungsleitungen richtet sich in erster Linie nach dem Außendurchmesser der in sie eingebetteten Kapillarmembranen. Daher weist die mindestens eine Versorgungsleitung bevorzugt einen Innendurchmesser im Bereich von 0,1 bis 10 mm auf. Ebenso ist bevorzugt, wenn die Wandstärke im Bereich von 0,1 bis 5 mm liegt. Im Falle der Verwendung einer Versorgungsleitung mit nicht-kreisförmigem Querschnitt wird als Innendurchmesser der äquivalente Durchmesser $d = 4A/U$ des Innenquerschnitts angesetzt mit A als der Fläche des Innenquerschnitts und U als dessen Umfang. Beispielsweise kann die Versorgungsleitung auch einen ovalen, oder näherungsweise quadratischen oder rechteckigen Innenquerschnitt aufweisen. Für die Versorgungsleitungen haben sich beispielsweise Silikonschläuche als geeignet erwiesen, durch deren Wand die Kapillarmembranenden hindurchtreten und in der sie eingeklebt sind. Vorzugsweise sind die Versorgungsleitungen aus einem flexiblen Silikonschlauch ausgebildet. Die Einbettung bzw. das Einkleben in die Wand der Versorgungsleitung kann mittels üblicher Kleber wie z.B. mittels härtbarer Silikonmaterialien, Polyurethanharzen oder einem Epoxidharzen erfolgen.

In einer vorteilhaften Ausführungsform kann das mindestens eine Kapillarmembransystem in Form einer Kapillarmembranmatte aus parallel zueinander angeordneten Kapillarmembranen ausgebildet sein, wobei die Kapillarmembranen in der Matte mittels zueinander beabstandeter und zueinander parallel verlaufender Verbindungselemente miteinander verbunden und durch die Verbindungselemente zueinander auf Abstand gehalten sind. Die Verbindungselemente können quer zu den parallel zueinander angeordneten Kapillarmembranen verlaufen oder auch unter einem anderen Winkel. Dabei berühren die Verbindungselemente die Kapillarmembranen an ihrem äußeren Umfang oder die umschlingen diese. Die Verbindungselemente weisen entlang ihrer Längserstreckung keine geschlossenen Strömungskanäle auf, sind also folglich entlang ihrer Längserstreckung nicht von Fluiden durchströmbar. Bei den Verbindungselementen kann es sich um Klebestreifen handeln oder beispielsweise auch um strangförmige Elemente aus einem Silikonmaterial. In einer bevorzugten Ausführungsform sind die Kapillarmembranen mittels garnförmiger Verbindungselemente zu einer Matte verbunden. Besonders bevorzugt handelt es sich bei den Verbindungselementen um textile Multifilamentgarne. Besonders bewährt haben sich als textile Multifilamentgarne multifile Polyestergarne, Polypropylengarne oder Polytetrafluorethylengarne. Bestens geeignet sind hydrophile Garne, vorzugsweise aus Polyester.

Bei der Kapillarmembranmatte kann es sich in einer bevorzugten Ausführungsform um eine Wirkmatte handeln. Bei solchen Wirkmatten sind die Kapillarmembranen und die Verbindungsfäden miteinander verwirkt und die Kapillarmembranen verlaufen quer zur Erstreckungsrichtung der Kapillarmembranmatte. Die Länge der Kapillarmembranen ist durch die Mattenbreite bestimmt. In einer weiteren bevorzugten Ausführungsform kann es sich bei der Kapillarmembranmatte um eine Webmatte handeln. Bei solchen Webmatten sind die Kapillarmembranen und die Verbindungsfäden miteinander verwoben. Die Kapillarmembranen verlaufen dabei in Erstreckungsrichtung oder Laufrichtung der Kapillarmembranmatte und die textilen Fäden quer dazu. Kapillarmembranwirkmatten und -webmatten sowie Möglichkeiten ihrer Herstellung werden beispielsweise in der DE 38 39 567, der DE 43 08 850 und in der EP 0 442 147 beschrieben. Insbesondere mittels der Wirktechnologie lassen sich auf einfache Weise Matten herstellen, bei denen die Kapillarmembranen an ihrem freien Ende U-förmig ausgebildet und dort verschlossen sind. Derartige Matten können durch mäanderförmige Ablage einer Kapillarmembran in zueinander parallele Stränge, die durch die Wirkfäden miteinander verbunden sind, hergestellt werden. Dabei werden nach Fertigstellung der Wirkmatte die U-förmig ausgebildeten Enden an mindestens einer Seite der Wirkmatte abgetrennt und die dabei entstehenden offenen Enden der Kapillarmembranen dann einer Versorgungsleitung eingebettet. Im Falle, dass die U-förmig ausgebildeten Enden an beiden Seite der Wirkmatte abgetrennt werden, können die entstehenden gegenüberliegenden offenen Enden jeweils in Versorgungsleitungen eingebettet werden.

In einer bevorzugten Ausgestaltung liegen die Kapillarmembranen innerhalb der Matte in einer solchen Dichte vor, dass der Abstand der Kapillarmembranen zueinander in der Matte das 1 bis 10-fache des Außendurchmessers der Kapillarmembranen beträgt, wobei der Abstand von den Längsachsen der Kapillarmembranen gemessen wird. Dabei sind Matten bevorzugt, in denen der Abstand der Kapillarmembranen zueinander in der Matte das 1,05 bis 6-fache des Außendurchmessers der Kapillarmembranen beträgt. Besonders bevorzugt sind Abstände der Kapillarmembranen zueinander in der Matte im Bereich des 1,05 bis 3-fachen des Außendurchmessers der Kapillarmembranen. In einer weiteren besonders bevorzugten Ausführungsform sind dabei Abstände der Kapillarmembranen zueinander in der Matte von mehr als dem 1,5 fachen des Außendurchmessers der Kapillarmembranen. Es wurde gefunden, dass sich hiermit eine sichere Trennung der Kapillarmembranen voneinander erreichen lässt.

Gleichzeitig kann es auch im Hinblick auf eine gute homogene Versorgung der zu behandelnden Wunde wichtig sein, dass die Kapillarmembranen in den Kapillarmembransystemen mittels mehrerer zueinander beabstandeter und zueinander parallel verlaufender Verbindungselemente miteinander zu einer Matte verbunden und durch die Verbindungselemente zueinander auf Abstand gehalten sind, und dass die Verbindungselemente sich zueinander in einem definierten Abstand befinden, der vorzugsweise im Bereich von 1 bis 50 mm liegt, wobei ein Abstand im Bereich von 3 bis 20 mm besonders bevorzugt und ein solcher im Bereich von 4 bis 6 mm bestens geeignet ist. Es hat sich nämlich gezeigt, dass die Kontaktstellen zwischen den Kapillarmembranen und den Verbindungselementen z.B. bei einer Versorgung der zu behandelnden Wunde z.B. mit einer Behandlungslösung oder einer Nährlösung in erheblichem Maße Verteilung der Flüssigkeit über die Fläche der Anordnung der Kapillarmembranen fördern. So wurde bei Auflage solcher Kapillarmembransysteme auf zu behandelnde Wunden beobachtet, dass es an den Kontaktstellen zu einer Begünstigung des Austritts von Flüssigkeit aus den Kapillarmembranen kommt.

Die Kapillarmembranen des mindestens einen Kapillarmembransystems weisen bevorzugt einen Außendurchmesser im Bereich von 200 bis 1500 $\mu$m auf. Ebenso sind Kapillarmembranen mit einer Wandstärke im Bereich von 20 bis 400 $\mu$m von Vorteil, wobei deren Außendurchmesser vorzugsweise in den zuvor genannten Bereichen liegen kann.

Bei dem vorliegenden Wundversorgungssystem kann das mindestens eine Kapillarmembransystem für die Zuführung bzw. Abführung von flüssigen Medien ausgelegt sein. Um dann eine gleichmäßige Versorgung der zu behandelnden Wunde zu gewährleisten, weisen in diesem Fall in einer bevorzugten Ausführungsform die Kapillarmembranen eine hohe Permeabilität für Flüssigkeiten auf. Vorzugsweise liegt hierbei der Transmembranfluss für Wasser der Kapillarmembranen im Bereich von 0,01 bis 50 ml/(min·cm$^2$·bar). Wie im späteren noch ausgeführt wird, ist es jedoch auch möglich, dass das Wundversorgungssystem Kapillarmembransysteme umfasst, welche beispielsweise der Zuführung von Sauerstoff zur Wunde oder der pH-Wert Regulierung dienen.

Die Kapillarmembranen des mindestens einen Kapillarmembransystems sind vorzugsweise bakteriendicht. Hierdurch kann gewährleistet werden, dass durch eine Zuführung z.B. von Spülflüssigkeiten und/oder anderen Behandlungslösungen keine Bakterien in die Wunde gelangen. Dabei wird - wie zuvor bereits erläutert - im Rahmen der vorliegenden Erfindung unter Bakteriendichtigkeit verstanden, dass die Kapillarmembranen eine nominelle Pore von 0,2 $\mu$m aufweisen. Bevorzugt weisen also die Kapillarmembranen des ersten und/oder des zweiten Kapillarmembransystems eine nominelle Pore von 0,2 $\mu$m auf.

Als Materialien für die Kapillarmembranen der Kapillarmembransysteme, aber auch für die semipermeable Membran des Filtermoduls kommen grundsätzlich alle im Stand der Technik bekannten organischen Polymere in Frage, die zur Ausbildung insbesondere von Kapillarmembranen geeignet sind, wobei diese Polymere eine gute Biokompatibilität aufweisen müssen. Darüber hinaus ist es auch erforderlich, dass das Membranpolymer eine Sterilisation des Wundversorgungssystems und des Filtermoduls beispielsweise über Dampfsterilisation, Sterilisation mittels $\gamma$-Strahlung oder Sterilisation mittels Ethylenoxid erlaubt. Dabei können die organischen Polymere natürliche Polymere sein oder Polymere, die auf synthetischen Wege hergestellt wurden. Natürliche Polymere sind insbesondere solche auf Basis von zellulosischen Polymeren, was Polymere, die sog. polymeranalogen Reaktionen unterzogen worden sind, ebenfalls umfasst. Beispiele für Polymere auf Basis von Zellulose sind solche aus regenerierter Zellulose, Zelluloseazetat oder modifizierter Zellulose wie z.B. Zelluloseester, Zelluloseäther, mit Benzylgruppen modifizierte Zellulose (Benzylzellulose) oder mit Diethylaminoethyl modifizierte Zellulose oder Mischungen dieser zellulosischen Polymere. Des Weiteren können auch Polymere auf der Basis von Chitin, bzw. Chitosan zum Einsatz kommen.

Als auf synthetischem Wege hergestellte Polymere, d.h. als synthetische Polymere können solche verwendet werden, die aus Polyolefinen, Polyamiden, Polyacrylnitrilen, Polycarbonaten, Polyestern oder Sulfonpolymeren sowie daraus gewonnen Modifikationen, Blends, Mischungen oder Copolymere dieser Polymere bestehen. Vorzugsweise werden solche verwendet, die auf Sulfonpolymeren, wie insbesondere Polysulfon oder Polyethersulfon, basieren. Den synthetischen Polymeren können weitere Polymere wie z.B. Polyethylenoxid, Polyhydroxyether, Polyethylenglykol, Polyvinylalkohol oder Polycaprolacton als Zusatzstoffe beigemischt werden. Die Kapillarmembranen können darüber hinaus noch eine Beschichtung mit einem Additiv aufweisen. Bevorzugt enthalten solche Kapillarmembranen ein Hydrophilierungsmittel, z.B. Polyvinylpyrrolidon oder auch hydrophile Modifikationen dieser Polymere.

Die Kapillarmembranen der Kapillarmembransysteme können mit Blick auf bestimmte Anwendungen z.B. über Ankopplung funktioneller Gruppen modifiziert sein oder beispielsweise mit Heparin oder einem Antibiotikum oder mehreren Antibiotika beschichtet sein.

Die Form der flächenförmig ausgebildeten Kapillarmembransysteme in ihrer flächigen Erstreckung kann beliebig sein. Im Fall von Kapillarmembransystemen aus zueinander parallelen Kapillarmembranen weisen die Kapillarmembransysteme in der einfachsten Ausführung eine quadratische oder rechteckige Form auf. Es ist jedoch z.B. bei Systemen, bei denen die Kapillarmembranen nur an einem ihrer Enden in eine Versorgungsleitung eingebettet sind, möglich, dass beispielsweise durch entsprechend angepasstes Abschweißen der freien, verschlossenen Enden der zueinander parallelen Kapillarmembranen eine bogenförmige Kontur ausgebildet wird. Ebenso ist es möglich, dass die Anordnung aus zueinander parallelen Kapillarmembranen z.B. auch eine trapezförmige Kontur aufweist.

Das vorliegende Wundversorgungssystem kann, wie bereits erwähnt, auch weitere Komponenten aufweisen wie z.B. mindestens eine weitere Anordnung von Kapillarmembranen. Bei den Kapillarmembranen der weiteren Anordnung kann es sich beispielsweise um Membranen zur Oxygenation handeln, d.h. Membranen, über die eine Zuführung von Sauerstoff zur Wunde möglich ist. Derartige Membranen werden beispielsweise in der in der EP-A-1 144 096, der EP-A-0 299 381 oder der DE-A-28 33 493 offenbart. Auch eine Kombination mit weiteren flächenförmig ausgebildeten Systemen bzw. Anordnungen von semipermeablen Kapillarmembranen oder fluidundurchlässigen Kapillaren ist möglich, über die z.B. eine Temperierung oder eine pH-Wert Regulierung erfolgen kann. Dabei können die jeweiligen Kapillarmembransysteme und eventuelle weitere flächenförmig ausgebildete Systeme von semipermeablen Kapillarmembranen oder fluidundurchlässigen Kapillaren aufeinander gelegt werden. Es ist jedoch auch möglich, dass z.B. die Kapillarmembranen unterschiedlicher Kapillarmembransysteme miteinander zu einer Matte verbunden sind, wobei die unterschiedlichen Kapillarmembranen mit ihren Enden in unterschiedliche Versorgungsleitungen eingebettet sind, die vorzugsweise an entgegengesetzten Seiten der Matte angeordnet sind. Solche Matten können beispielsweise durch Verwirken von zueinander versetzt angeordneten, mäanderförmig abgelegten Kapillarmembranen erhalten werden, bei denen die U-förmigen Umlenkungen der Kapillarmembranen sich an unterschiedlichen Positionen über der Mattenbreite befinden. Durch Schneiden der jeweils außenliegenden U-förmigen Umlenkungen werden die Kapillarmembranen an jeweils nur einer Seite der Matte geöffnet und können dort in eine Versorgungsleitung eingebettet werden.

In der bevorzugten Ausführungsform, in der das System zur Wundbehandlung bzw. das Wundversorgungssystem neben dem ersten Kapillarmembransystem und gegebenenfalls einem zweiten Kapillarmembransystem ein Drainagesystem umfasst, mittels dessen eine Abführung z.B. von Spülflüssigkeit oder von Exsudat aus der Wunde möglich ist, kann das Drainagesystem einen Saugschwamm umfassen, welcher über eine Absaugleitung für die Spülflüssigkeit und/oder Exsudat verfügt. Das Drainagesystem kann auch als ein weiteres Kapillarmembransystem ausgebildet sein. Vorzugsweise handelt es sich bei dem Drainagesystem jedoch um mindestens einen Drainagekatheter, z.B. in Gestalt eines Schlauchstücks, beispielsweise aus einem Silikonmaterial, oder eines Röhrchens. Ein solcher Drainagekatheter kann in seiner Wand Perforierungen aufweisen, über die nach Anschluss des Drainagekatheders an eine Unterdruckeinheit Flüssigkeiten aus der Wunde abgesaugt werden können. Der mindestens eine Drainagekatheter weist bevorzugt einen Innendurchmesser im Bereich von 0,1 bis 15 mm und eine Wandstärke im Bereich von 0,1 bis 3 mm auf. Der Drainagekatheter kann auch einen nicht-kreisförmigen Querschnitt aufweisen. In diesem Fall wird als Innendurchmesser der äquivalente Durchmesser $d_D = 4A_D/U_D$ des Innenquerschnitts angesetzt mit $A_D$ als der Fläche des Innenquerschnitts des Drainagekatheters und $U_D$ als dessen Umfang.

In einer Ausführungsform kann das Wundversorgungssystem des Weiteren eine taschenförmige Wundauflage umfassen, wobei die taschenförmige Wundauflage an ihrem äußeren Rand geschlossen ist und eine Oberseite, eine Unterseite und ein Tascheninneres aufweist, wobei die Unterseite und die Oberseite jeweils aus einem flächigen Material ausgebildet sind und die Unterseite permeabel für Fluide ist, und wobei das erste und die gegebenenfalls vorhandenen weiteren Kapillarmembransysteme im Tascheninneren angeordnet sind. Hierbei liegt die Verbindung der Versorgungsleitungen mit dem Filtermodul und eventuell vorhandenen Aufnahmebehältern usw. außerhalb der taschenförmigen Wundauflage.

Die taschenförmige Wundauflage mit den darin enthaltenen Kapillarmembransystemen kann so in eine zu behandelnde Wunde eingelegt werden, dass die Unterseite mit der Wunde in Kontakt ist. Über die Kapillarmembransysteme können der Wunde die gewünschten Flüssigkeiten zugeführt werden, die sich nach Austritt aus den Kapillarmembranen in der Tasche verteilen und über die semipermeable Taschenunterseite an die Wunde abgegeben werden.

Die taschenförmige Wundauflage kann vorzugsweise so ausgeführt sein, dass sich die Verbindung der Kapillarmembranen der Kapillarmembransysteme mit der jeweiligen Versorgungsleitung im Tascheninneren befindet und die jeweiligen Versorgungsleitungen über an ihren äußeren Querschnitt fluiddicht angepasste Durchtrittsöffnungen aus der taschenförmigen Wundauflage herausführen. Ebenso kann die Verbindung der Kapillarmembranen mit der jeweiligen Versorgungsleitung außerhalb der taschenförmigen Wundauflage auf der Oberseite angeordnet sein und die Anordnung der Kapillarmembranen zur Verbindung mit der mindestens einen Versorgungsleitung über eine fluiddicht angepasste Durchtrittsöffnung aus der taschenförmigen Wundauflage herausführen.

Im Falle, dass das Wundversorgungssystem eine taschenförmigen Wundauflage umfasst, erstrecken sich die Kapillarmembransysteme flächig im Tascheninneren. Die Dimensionen der Kapillarmembransysteme ergeben sich jeweils aus deren äußeren Abmessungen in der flächigen Erstreckung. Vorzugsweise füllt das mindestens eine Kapillarmembransystem hinsichtlich seiner flächigen Erstreckung das Tascheninnere der taschenförmigen Wundauflage in dessen flächiger Erstreckung mindestens zu 20 % und besonders bevorzugt mindestens zu 50 % aus. Von besonderem Vorteil ist es, wenn das mindestens eine Kapillarmembransystem hinsichtlich seiner flächigen Erstreckung das Tascheninnere der taschenförmigen Wundauflage in dessen flächiger Erstreckung mindestens zu 70 % ausfüllt, wobei auch Füllgrade im Bereich von 90 % realisiert werden können. Dabei ist es von Vorteil, wenn das mindestens eine Kapillarmembransystem in der taschenförmigen Wundauflage mittig angeordnet sind.

Die taschenförmige Wundauflage kann beliebige Konturen aufweisen. Vorzugsweise ist die Kontur jedoch rund, oval, quadratisch oder rechteckig. Unterseite und Oberseite der taschenförmigen Wundauflage sind am äußeren Rand bzw. an den äußeren Kanten der Wundauflage beispielsweise durch Verschweißen oder Verkleben miteinander verbunden. Zur Verklebung sind u.a. Silikonstreifen geeignet, die ausgehärtet werden. Bei rechteckigen oder bei quadratischen taschenförmigen Wundauflagen weisen darin angeordnete flächenförmig ausgebildete Kapillarmembransysteme vorzugsweise ebenfalls eine rechteckige oder quadratische Kontur auf. Bei runden oder ovalen taschenförmigen Wundauflagen sind darin befindliche Kapillarmembransysteme zweckmäßigerweise ebenfalls quadratisch oder rechteckig ausgebildet, wobei hinsichtlich der Abmessungen ebenfalls die zuvor genannten Dimensionen gelten. Sie können jedoch auch an die Kontur der taschenförmigen Wundauflage angepasst sein, beispielsweise durch entsprechend angepasstes Abschweißen der nicht eingebetteten Enden der Kapillarmembranen bei Kapillarmembransystemen mit jeweils nur einer Versorgungsleitung, so dass sich an dieser Kante der Kapillarmembransysteme eine bogenförmige Kontur ergibt.

Die Unterseite der taschenförmigen Wundauflage ist gegenüber Fluiden durchlässig, d.h. permeabel. Dabei kann die Unterseite beispielsweise aus einem vliesförmigen, flächigen Material, einem gitterförmigen oder netzartigen Material, einer perforierten Folie oder einer semipermeablen mikroporösen Flachmembran bestehen. In einer vorteilhaften Ausführungsform besteht die Unterseite aus einem vliesförmigen, flächigen Material oder einer semipermeablen mikroporösen Flachmembran. Die Unterseite weist bevorzugt eine Permeabilität für Wasser von mindestens 0,01 ml/(min·cm$^2$·bar) und besonders bevorzugt von mindestens 10 ml/(min·cm$^2$·bar) auf. Bestens bewährt hat sich eine Unterseite mit einer Permeabilität für Wasser von mindestens 500 ml/(min·cm$^2$·bar).

Für die vorgesehenen Anwendungen des Wundversorgungssystems, bei denen der Wunde über das Wundversorgungssystem nicht nur Flüssigkeit zugeführt wird, sondern auch eine Entsorgung, d.h. Abführung von Flüssigkeiten aus der Wunde über das Drainagesystem erfolgen soll, ist es von Vorteil, wenn in der Unterseite Öffnungen vorhanden sind, wobei die Öffnungen vorzugsweise einen Durchmesser von mindestens 100 μm aufweisen. Dabei sind Durchmesser der Öffnungen von höchstens 10 mm bevorzugt und von höchstens 5 mm besonders bevorzugt. Im Falle, dass die Unterseite aus einer semipermeablen mikroporösen Flachmembran besteht, weist diese in einer vorteilhaften Ausführungsform zusätzlich Öffnungen z.B. in Form von Perforationen auf. Im Falle, dass die Öffnungen eine nicht-kreisförmige Kontur aufweisen, wird als Durchmesser der äquivalente Durchmesser D=4A/U der Öffnung angesetzt mit A als der Fläche der jeweiligen Öffnung und U als deren Umfang. Die Öffnungen können regelmäßig oder unregelmäßig über die Fläche der Unterseite verteilt sein, wobei eine regelmäßige, homogene Verteilung bevorzugt ist. Dabei kann der Abstand zwischen den Öffnungen im Bereich von 1 bis 20 mm liegen, gemessen von äußeren Rand der Öffnungen.

Unter- und die Oberseite der taschenförmigen Wundauflage können aus gleichen oder unterschiedlichen Materialien bestehen. Während die Unterseite jedoch stets gegenüber Flüssigkeiten durchlässig ist, ist die Oberseite bevorzugt aus einem fluidundurchlässigen, vorzugsweise folienförmigen Material ausgebildet, das an seiner bzw. seinen Seitenkanten fluiddicht mit der Unterseite verbunden ist. Es kann sich bei der Oberseite auch um eine semipermeable, mikroporöse Flachmembran handeln. In diesem Fall weist die Oberseite jedoch eine geringere Permeabilität gegenüber Fluiden auf als die Unterseite, um in der Anwendung eine Verteilung zugeführter Flüssigkeit auf der Unterseite der taschenförmigen Wundauflage und damit zur Wunde hin zu gewährleisten. Im Falle, dass es sich bei Unterseite und Oberseite um dieselbe oder die gleiche semipermeablen mikroporösen Flachmembran handelt, weist die Unterseite Perforationen auf.

Als Materialien für die Unter- bzw. die Oberseite der taschenförmigen Wundauflage kommen grundsätzlich dieselben organischen Polymere in Frage, die zuvor als Polymere für die Kapillarmembranen genannt wurden und die sich zu Flachfolien bzw. Flachmembranen verarbeiten lassen. Vorzugsweise sind Unter- und/oder Oberseite der taschenförmigen Wundauflage aus Polyolefinen, Polyamiden, Polyacrylnitril, Polycarbonaten Polyester oder Sulfonpolymeren sowie daraus gewonnen Modifikationen, Blends, Mischungen oder Copolymere dieser Polymere aufgebaut. Besonders bevorzugt umfassen Unter- und Oberseite als Material Sulfonpolymere, wobei Polysulfon oder Polyethersulfon bestens geeignet sind.

Das gemäß einer Ausführungsform vorhandene Drainagesystem, vorzugsweise in Gestalt mindestens eines Drainagekatheters, führt im bevorzugten Fall, dass das Wundversorgungssystem eine taschenförmige Wundauflage umfasst, über eine entsprechend fluiddicht angepasste Durchtrittsöffnung aus der taschenförmigen Wundauflage heraus und ist außerhalb der taschenförmigen Wundauflage mit einer Unterdruckeinheit verbindbar, um so in der Anwendung einen Unterdruck im Tascheninneren zu erzeugen. Bei dem mindestens einen Drainagekatheder kann es sich um ein Schlauchstück, beispielsweise aus einem Silikonmaterial, oder um ein Röhrchen handeln, das im Tascheninneren der Wundauflage angeordnet ist und über die Durchtrittsöffnung aus der Wundauflage herausführt. An dem sich im Inneren der taschenförmigen Wundauflage befindlichen Segment des mindestens einen Drainagekatheters weist dieser vorzugsweise in seiner Wand Perforierungen auf, über die nach Anschluss des mindestens einen Drainagekatheders an eine Unterdruckeinheit Flüssigkeiten wie z.B. auch Exsudat oder zugeführtes Serum aus der Wunde bzw. aus dem Inneren der taschenförmigen Wundauflage und aus der Wunde abgesaugt werden kann.

In einer bevorzugten Ausführungsform sind erstes und zweites Kapillarmembransystem des Wundversorgungssystems identisch und bilden ein einziges Versorgungs-Kapillarmembransystem aus, welches mit mindestens einer Versorgungsleitung verbunden ist, und die Vorrichtung zur Gewinnung von Serum und der mit dem zweiten Kapillarmembransystem verbundene Entnahmebehälter sind über die mindestens eine Versorgungsleitung des Versorgungs-Kapillarmembransystems mit dem Versorgungs-Kapillarmembransystem verbunden. Dabei kann das Versorgungs-Kapillarmembransystem zwei Versorgungsleitungen aufweisen, die sich an den gegenüberliegenden Enden seiner Kapillarmembranen befinden. In diesem Fall können z.B. die Vorrichtung zur Gewinnung von Serum und der Entnahmebehälter, der vorzugsweise eine Spülflüssigkeit enthält, mit unterschiedlichen und voneinander getrennten Versorgungsleitungen verbunden sein. Es ist jedoch auch möglich, dass die Vorrichtung zur Gewinnung von Serum und der Entnahmebehälter über z.B. mittels eines T-Stücks oder Y-Konnektors miteinander verbundene Leitungsabschnitte mit nur einer Versorgungsleitung des Versorgungs-Kapillarmembransystems verbunden sind. Hierbei sind auch Ausführungen des Versorgungs-Kapillarmembransystems möglich, bei dienen das Versorgungs-Kapillarmembransystem mit nur einer Versorgungsleitung verbunden ist.

Für die Anwendung ist bevorzugt, wenn z.B. die Spülflüssigkeit aus dem Entnahmebehälter und das Serum aus dem Filtermodul alternierend zugeführt werden können. Bevorzugt weist hierzu der Leitungsweg zwischen dem Filtermodul und dem mit dem Filtermodul verbundenen Kapillarmembransystem ein dem Filtermodul zugeordnetes erstes Absperrorgan auf. Ebenso weist der Leitungsweg zwischen dem genannten Entnahmebehälter und dem mit dem Entnahmebehälter verbundenen Kapillarmembransystem ein dem Entnahmebehälter zugeordnetes zweites Absperrorgan auf, wobei erstes und zweites Absperrorgan unabhängig voneinander einstellbar sind. Je nach Ausführung des Wundversorgungssystems können die Absperrorgane am Filtermodul bzw. am Entnahmebehälter selbst, in den Leitungsabschnitten zwischen Filtermodul bzw. Entnahmebehälter und einem die Leitungsabschnitte von Filtermodul und Entnahmebehälter verbindenden T-Stück oder Y-Konnektor oder in der jeweiligen Versorgungsleitung angebracht sein.

In einer bevorzugten Ausführungsform sind Filtermodul und Entnahmebehälter über Male-/Female-Konnektoren lösbar mit der jeweiligen Versorgungsleitung verbunden. Besonders bevorzugt sind Male-/Female-Konnektoren Luer-Lock-Konnektoren. Auch hier kann die Verbindung als sterile Schweißverbindung ausgeführt sein, wie sie mittels der zuvor genannten Sterilkonnektoren hergestellt werden kann.

Als Spülflüssigkeit, wie sie, wie angegeben, über das zweite Kapillarmembransystem zugeführt werden kann, kommen übliche Flüssigkeiten in Frage, die für die Wundreinigung geeignet sind. Bevorzugt enthält der mit dem zweiten Kapillarmembransystem verbundene Entnahmebehälter eine KochsalzLösung. Beispielsweise über weitere Kapillarmembransysteme können zusätzliche Behandlungslösungen der Wunde zugeführt werden wie z.B. Lösungen mit Wachstumsfaktoren, Antibiotika oder andere Medikamente enthaltende Lösungen, Lösungen zur pH-Wert Regulierung.

Für die Charakterisierung der Eigenschaften der im Wundversorgungssystem eingesetzten Kapillarmembranen bzw. Flachmembranen werden die folgenden Messmethoden zu Grunde gelegt:

Transmembranfluss (Wasserpermeabilität) für Kapillarmembranen:

Aus den zu prüfenden Kapillarmembranen wird eine Prüfzelle mit definierter Kapillarmembranzahl und Länge gefertigt. Die Kapillarmembranen werden dafür beidseitig an ihren Enden in ein Polyurethanharz eingebettet. Nach dem Aushärten des Harzes werden die Einbettungen auf eine Länge von ca. 30 mm geschnitten, wobei die Lumina der Kapillarmembranen durch den Schnitt geöffnet werden. Die Kapillarlumina in den Einbettungen müssen auf Durchgängigkeit überprüft werden. Die freie Länge der Kapillarmembranen zwischen den Einbettungen beträgt üblicherweise 120 +/- 10 mm. Die Anzahl der Kapillarmembranen ist so zu bemessen, dass unter Berücksichtigung der freien Länge und des Innendurchmessers der Kapillarmembranen eine Filtrationsfläche von ca. 30 cm$^2$ in der Prüfzelle bereitgestellt wird.

Die Prüfzelle wird mit in eine Prüfapparatur eingebunden und mit auf 25°C temperiertem ultrafiltriertem und vollentsalztem Wasser bei einem definiertem Prüfdruck (ca. 0,4 bar) durchströmt. Die während einer Messzeit von 2 min erhaltene filtrierte Wassermenge, d.h. das während der Messung erzeugte Permeat wird gravimetrisch oder volumetrisch erfasst. Vor Beginn der Messung muss die Anlage luftfrei gespült werden. Zur Bestimmung des TMF werden in der Prüfapparatur der Eingangs- und Ausgangsdruck an der Prüfzelle gemessen. Die Messung wird bei 25°C durchgeführt.

Der Transmembranfluss TMF wird nach der Formel (I)

$$TMF = \frac{V_W}{\Delta t \cdot A_M \cdot \Delta p} \quad \left[ \frac{ml}{cm^2 \cdot min \cdot bar} \right] \quad (I)$$

ermittelt. Hierbei sind:

$V_W$ = durch die Membranprobe während der Messzeit hindurch geströmte Wasservolumen [ml]
$\Delta t$ = Messzeit [min]
AM = durchströmte Fläche der Membranprobe (üblicherweise 30 cm$^2$)
$\Delta p$ = eingestellter Druck während der Messung [bar]

Permeabilität für Wasser der Unterseite der taschenförmigen Wundauflage:

Aus dem zu prüfenden flächigen Material der Unterseite der taschenförmigen Wundauflage werden scheibenförmige Proben ausgestanzt und in einen geeigneten Probenhalter am Umfang fluiddicht so eingespannt, dass eine freie Messfläche von 17,35 cm$^2$ resultiert. Der Probenhalter befindet sich in einem Gehäuse, das von Wasser druckbeaufschlagt durchströmt werden kann. Die eingespannte Probe wird dann von auf 25°C temperiertem, vollentsalztem Wasser unter einem definierten Druck zwischen 0,1 und 0,2 bar durchströmt. Es wird das während einer Messzeit von 60 s durch die Probe hindurch geströmte Wasservolumen gravimetrisch oder volumetrisch ermittelt.

Die Permeabilität für Wasser TMF$_W$ wird nach der Formel (II)

$$TMF_W = \frac{V_W}{\Delta t \cdot A_M \cdot \Delta p} \quad \left[ \frac{ml}{cm^2 \cdot min \cdot bar} \right] \quad (II)$$

ermittelt. Hierbei sind:

$V_W$ = durch die Probe während der Messzeit hindurch geströmte Wasservolumen [ml]
$\Delta t$ = Messzeit [min]
AM = durchströmte Fläche der Probe (17,35 cm$^2$)
$\Delta p$ = eingestellter Druck während der Messung [bar]

Die Erfindung wird anhand der folgenden Figuren näher erläutert, wobei durch die Figuren der Umfang der Erfindung nicht eingeschränkt wird:

Es zeigen:

Fig. 1: Wundversorgungssystem mit einer einlagigen Matte aus Kapillarmembranen und Versorgungsleitungen an beiden Enden der Matte.

Fig. 2: Wundversorgungssystem mit einer Versorgungsleitung an einem der Mattenenden sowie U-förmig ausgebildeten Kapillarmembranenden am gegenüberliegenden Mattenende.

Fig. 3: Querschnitt (schematisch) durch ein im System zur Wundbehandlung einsetzbares taschenförmiges Wundauflagensystem.

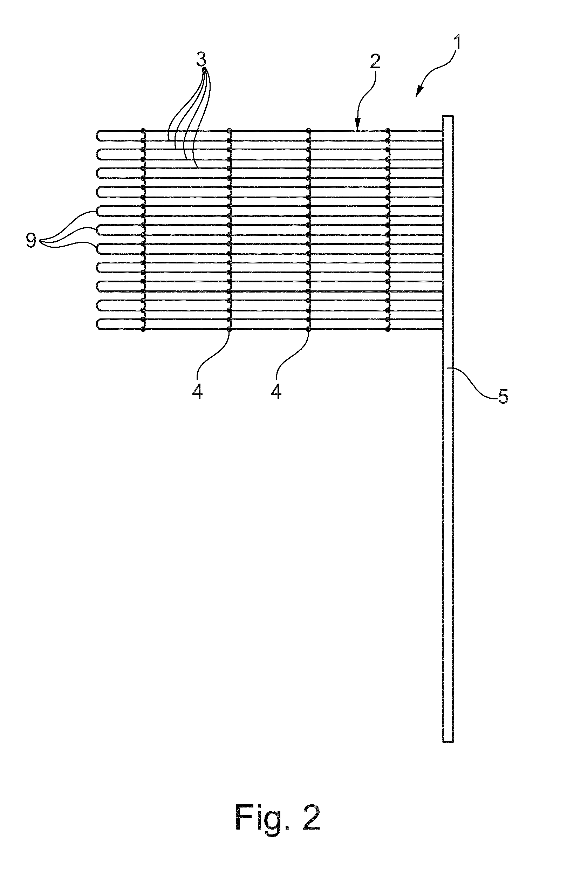

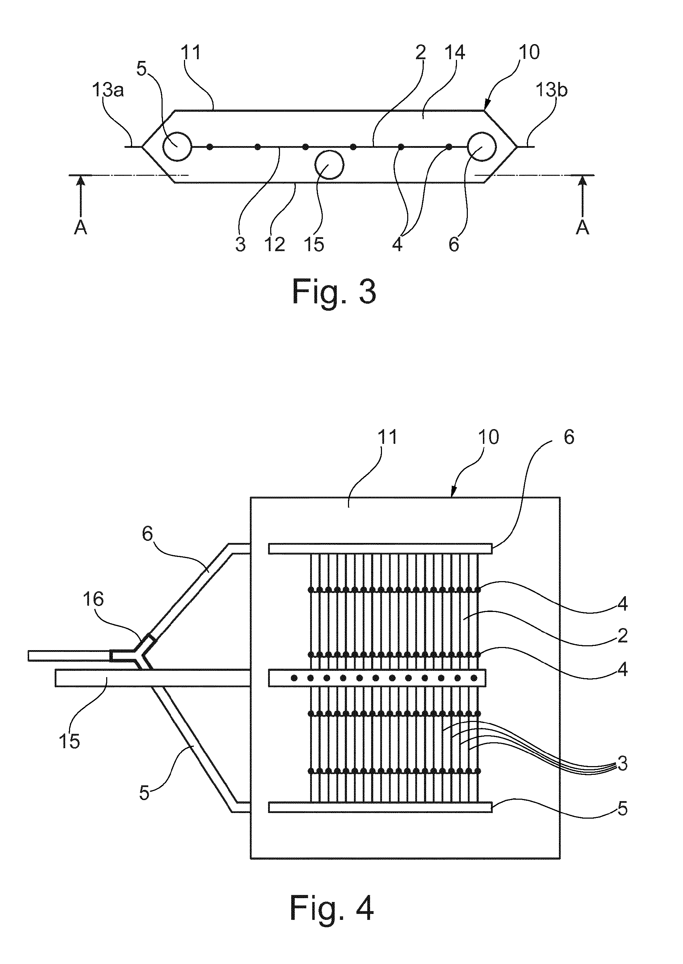

Fig. 2:     Schnitt A - A des in Figur 3 im Querschnitt dargestellten taschenförmigen Wundauflagesystems.

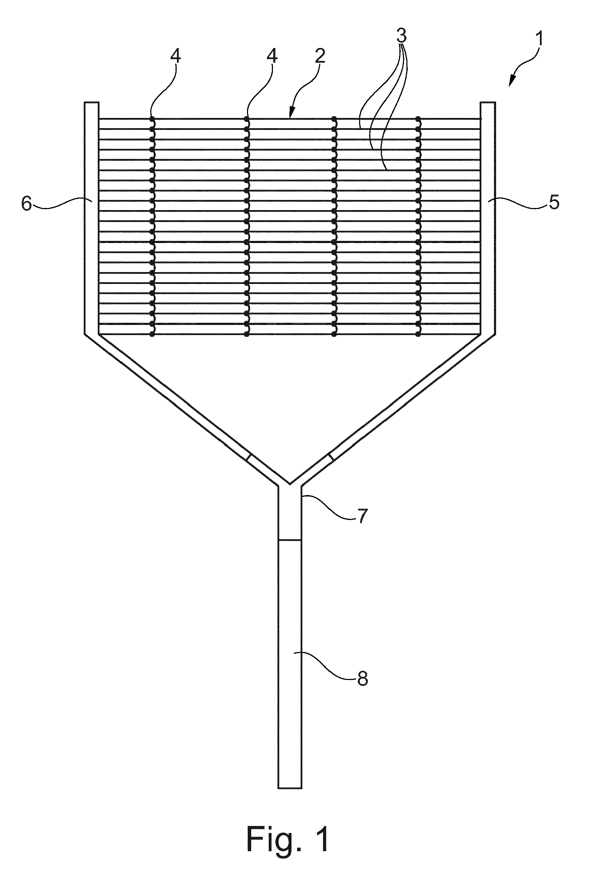

Figur 1 zeigt in der Draufsicht schematisch und nicht maßstäblich ein im erfindungsgemäßen System zur Wundbehandlung verwendbares Wundversorgungssystem 1 mit einem Kapillarmembransystem 2 aus Kapillarmembranen 3. Die Kapillarmembranen 3 sind mittels zueinander parallel verlaufender Verbindungselemente 4 zu einer Matte so verbunden, dass sie zueinander parallel angeordnet und zueinander auf Abstand gehalten sind. Die Kapillarmembranen 3 sind im vorliegenden Beispiel mit ihren gegenüber liegenden Enden so in Versorgungsleitungen 5, 6 eingebettet, dass zwischen den Lumen der Versorgungsleitungen 5,6 und dem Lumen der Kapillarmembranen 3 eine Fluidverbindung besteht. Die Versorgungsleitungen 5, 6 sind über ein Y-Stück 7 zu einer gemeinsamen Leitung 8 zusammengefasst. Aus diesem Aufbau ergibt sich, dass z.B. das Serum, das über die Leitung 8 zugeführt wird, auf die Versorgungsleitungen 5, 6 aufgeteilt wird und den Kapillarmembranen 3 im dead-end Modus zugeführt wird. Das Serum strömt über die porösen, semipermeablen Wände der Kapillarmembranen 3 aus diesen aus und wird über der Fläche der Anordnung 2 aus Kapillarmembranen 3 gleichmäßig der Wunde zugeführt.

Figur 2 zeigt ebenfalls schematisch und in nicht-maßstäblicher Darstellung ein Wundversorgungssystem 1, bei dem die Kapillarmembranen 3 nur mit einer Versorgungsleitung 5 verbunden sind. Die Kapillarmembranen sind an ihren beiden Enden offen und mit ihren beiden Enden in eine Versorgungsleitung 5 eingebettet. Die freien Enden 10 der Kapillarmembranen 3 sind an dem der Versorgungsleitung 5 gegenüberliegenden Ende der Matte U-förmig ausgebildet und dadurch dort geschlossen. Auf diese Weise erfolgt bei den Kapillarmembranen 3 des in Figur 3 gezeigten Wundversorgungssystems 1 die Anströmung im dead-end Modus.

Figur 3 zeigt schematisch einen Querschnitt durch eine taschenförmige Wundauflage 10, welche eine Oberseite 11 und eine Unterseite 12 aufweist, die an ihrem Rand 13a, 13b z.B. miteinander verschweißt sind, wodurch ein geschlossenes Tascheninneres 14 entsteht. Im Tascheninneren 14 ist vorliegend ein erstes ein flächenförmiges Kapillarmembransystem 2 angeordnet, welches Kapillarmembranen 3 umfasst, die über zueinander parallel verlaufende Verbindungselemente 4 vorzugsweise in Gestalt von Multifilamentgarnen miteinander verbunden und zueinander auf Abstand gehalten sind. Ein entsprechend der vorliegenden Erfindung gegebenenfalls erforderliches zweites Kapillarmembransystem kann in einer Ausführungsform oberhalb oder unterhalb des dargestellten Kapillarmembransystems 2 angeordnet sein, wobei die Versorgungsleitungen des ersten Kapillarmembransystems und des zweiten Kapillarmembransystems dann auf der gleichen Seite oder auf unterschiedlichen Seiten der taschenförmige Wundauflage 10 aus dieser herausgeführt werden können.

Die Kapillarmembranen 3 münden im vorliegenden Fall mit ihren gegenüberliegenden Enden in Versorgungsleitungen 5, 6, so dass durch die Versorgungsleitungen 5, 6 und das Kapillarmembransystem 2 Flüssigkeiten, Medien, Gase und/oder andere Stoffe hindurchleitbar sind. Die Versorgungsleitungen 5, 6 werden durch die Oberseite 11 der taschenförmigen Wundauflage 10 herausgeführt (hier nicht dargestellt).

Unterhalb des flächenförmigen Kapillarmembransystems 2 ist ein Drainageschlauch 15 angeordnet, über den z.B. sich in der Wunde ansammelndes Exsudat entfernt werden kann.

Figur 4 zeigt das in Figur 3 dargestellte Wundauflagesystem in einem Querschnitt entlang der Linie A - A. Im Prinzip handelt es sich um eine Draufsicht, von einer Position oberhalb der Unterseite 12 der taschenförmigen Wundlauflage 10 in Richtung der Oberseite 11 der taschenförmigen Wundlauflage 10. Unterhalb der Oberseite 11 , d.h. wie in Figur 3 dargestellt, zwischen Unterseite 12 und Oberseite 11 ist das Kapillarmembransystem 2 angeordnet, das aus zueinander parallelen Kapillarmembranen 3 aufgebaut ist, die über die Verbindungselemente 4 miteinander verbunden und zueinander auf Abstand gehalten sind. Die Kapillarmembranen 3 sind mit ihren gegenüberliegenden Enden in die Versorgungsleitungen 5, 6 eingebettet, so dass durch die Versorgungsleitungen 5, 6 und das Kapillarmembransystem 2 Flüssigkeiten, Medien, Gase und/oder andere Stoffe hindurchleitbar sind. Die Versorgungsleitungen 5, 6 werden durch die Oberseite 11 der taschenförmigen Wundauflage 10 durch entsprechend angepasste Öffnungen in der Oberseite 11 aus der taschenförmigen Wundauflage 10 herausgeführt und im vorliegenden Beispiel über einen Y-Verbinder 16 außerhalb der taschenförmigen Wundauflage 10 zusammengeführt. Im vorliegenden Fall wird also das Kapillarmembransystem 2 im Dead-end Modus betrieben, d.h. ein über die Versorgungsleitungen 5, 6 zugeführtes Medium wird in das Kapillarmembransystem 2 eingeleitet und tritt vollständig über die Wände der Kapillarmembranen 3 in das Tascheninnere ein.

In der Figur 2 ist auch der Drainageschlauch 15 dargestellt, der unterhalb des Kapillarmembransystems 2 angeordnet ist. Der Drainageschlauch weist in seiner Wand Perforationen auf, so dass z.B. sich in der Wunde ansammelndes Exsudat über den Drainageschlauch angesaugt und so aus der Wunde entfernt werden kann. Der Drainageschlauch 15 wird ebenfalls über eine entsprechend angepasste Öffnung in der Oberseite 11 aus der taschenförmigen Wundauflage 10 herausgeführt und ist z.B. mit einer (nicht dargestellten) Unterdruckeinheit verbindbar.

**Patentansprüche**

1. System zur Wundbehandlung mit Serum mit einer Vorrichtung zur Gewinnung von Serum aus Vollblut, wobei die Vorrichtung umfasst:

   - einen Blutbeutel zur Aufnahme von Vollblut und zur Koagulation des Vollbluts in einen die Zellbestandteile des Bluts enthaltenden Feststoffanteil und in eine Serum enthaltene Flüssigkeit, wobei der Blutbeutel einen Auslass zur Ableitung der Flüssigkeit aus dem Blutbeutel aufweist und im Bereich des Auslasses eine Sperre, welche zum Rückhalt zumindest eines Großteils des Feststoffanteils geeignet ist,
   - einen mit dem Blutbeutel über den Auslass des Blutbeutels in Fluidverbindung stehenden Filtermodul,

     - wobei der Filtermodul ein Gehäuse mit einem Innenraum und einer den Innenraum begrenzenden Innenwand aufweist, in dem eine semipermeable Membran angeordnet ist, die den Innenraum in einen Retentatraum und einen Permeatraum unterteilt,
     - wobei die semipermeable Membran eine Auftrennung der aus dem Blutbeutel abgeleiteten Flüssigkeit in ein Permeat, welches das Serum umfasst, und in ein Retentat, in dem eventuell in der Flüssigkeit enthaltene partikulärer Bestandteile verbleiben, ermöglicht und
     - wobei der Filtermodul eine Einlasseinrichtung zum Einleiten der aus dem Blutbeutel abgeleiteten Flüssigkeit in den Retentatraum, einen Retentatauslass zum Ableiten von Retentat aus dem Retentatraum und einen Permeatauslass zum Ableiten des Permeats aus dem Permeatraum aufweist, und

   wobei das System zur Wundbehandlung des Weiteren ein Wundversorgungssystem zum Ausbringen des Permeats auf eine Wunde umfasst, welches ein erstes flächenförmig ausgebildetes Kapillarmembransystem aufweist, wobei das erste Kapillarmembransystem mit mindestens einer Versorgungsleitung verbunden ist, so dass durch die Versorgungsleitung und das erste Kapillarmembransystem Flüssigkeiten hindurchdurchleitbar und einer Wunde zuführbar sind, und wobei der Permeatauslass über eine die mindestens eine Versorgungsleitung des ersten Kapillarmembransystems umfassende Permeatleitung mit dem ersten Kapillarmembransystem verbunden ist.

2. System zur Wundbehandlung nach Anspruch 1, **dadurch gekennzeichnet dass** es des Weiteren ein zweites flächenförmig ausgebildetes Kapillarmembransystem umfasst, welches mit mindestens einer Versorgungsleitung verbunden ist, so dass durch die Versorgungsleitung und das zweite Kapillarmembransystem Flüssigkeiten, Medien, Gase und/oder andere Stoffe durchleitbar sind, sowie einen Entnahmebehälter, welcher über einen Leitungsweg, der mindestens eine der Versorgungsleitungen des zweiten Kapillarmembransystems umfasst, mit dem zweiten Kapillarmembransystem lösbar verbunden ist und über den dem zweiten Kapillarmembransystem eine Spülflüssigkeit zuführbar ist.

3. System zur Wundbehandlung nach Anspruch 1 oder 2, **dadurch gekennzeichnet, dass** es ein Drainagesystem umfasst, welches mit einer Unterdruckeinheit koppelbar ist und über das Flüssigkeiten aus der zu behandelnden Wunde abführbar sind.

4. System zur Wundbehandlung nach einem oder mehreren der Ansprüche 1 bis 3, **dadurch gekennzeichnet dass** die semipermeable Membran des Filtermoduls ein Bündel von Hohlfasermembranen ist und der Retentatraum durch die Lumina der Hohlfasermembranen und der Permeatraum durch den die Hohlfasermembranen umgebenden und durch die Innenwand des Gehäuses begrenzten Außenraum ausgebildet wird.

5. System zur Wundbehandlung nach Anspruch 4, **dadurch gekennzeichnet, dass** das Bündel von Hohlfasermembranen U-förmig ausgebildet ist.

6. System zur Wundbehandlung nach einem oder mehreren der Ansprüche 1 bis 5, **dadurch gekennzeichnet, dass** die semipermeable Membran des Filtermoduls einen Transmembranfluss im Bereich von 10.000 bis 40.000 l/(m$^2$·h·bar) aufweist.

7. System zur Wundbehandlung nach einem oder mehreren der Ansprüche 1 bis 6, **dadurch gekennzeichnet, dass** die semipermeable Membran des Filtermoduls eine nominelle Pore von 0,2 μm aufweist.

8. System zur Wundbehandlung nach Anspruch 1 oder 2, **dadurch gekennzeichnet, dass** das erste und/oder das zweite Kapillarmembransystem in Form einer Matte aus parallel zueinander angeordneten Kapillarmembranen ausgebildet ist, wobei die Kapillarmembranen in der Matte mittels zueinander beabstandeter und zueinander parallel verlaufender Verbindungselemente miteinander verbunden und durch die Verbindungselemente zueinander auf Abstand gehalten sind.

9. System zur Wundbehandlung nach Anspruch 1 oder 2, **dadurch gekennzeichnet, dass** die Kapillarmembranen des ersten bzw. des zweiten Kapillarmembransystems mit mindestens einem ihrer Enden in jeweils einer einzelnen Versorgungsleitung eingebettet sind.

10. System zur Wundbehandlung nach Anspruch 1 oder 2, **dadurch gekennzeichnet, dass** das erste und/oder das zweite Kapillarmembransystems mit jeweils zwei Versorgungsleitungen verbunden ist, wobei die Kapillarmembranen des jeweiligen Kapillarmembransystems mit ihren gegenüberliegenden Enden in jeweils eine Versorgungsleitung eingebettet sind.

11. System zur Wundbehandlung nach Anspruch 1 oder 2, **dadurch gekennzeichnet, dass** die Kapillarmembranen des ersten und/oder des zweiten Kapillarmembransystems einen Transmembranfluss für Wasser im Bereich von 0,01 bis 50 ml/(min·cm$^2$·bar) aufweisen.

12. System zur Wundbehandlung nach Anspruch 1 oder 2, **dadurch gekennzeichnet, dass** die Kapillarmembranen des ersten und/oder des zweiten Kapillarmembransystems eine nominelle Pore von 0,2 $\mu$m aufweisen.

13. System zur Wundbehandlung nach einem oder mehreren der Ansprüche 1 bis 12, **dadurch gekennzeichnet, dass** es des Weiteren eine taschenförmige Wundauflage umfasst, wobei die taschenförmige Wundauflage an ihrem äußeren Rand geschlossen ist und eine Oberseite, eine Unterseite und ein Tascheninneres aufweist, wobei die Unterseite und die Oberseite jeweils aus einem flächigen Material ausgebildet sind und die Unterseite permeabel für Fluide ist und wobei das erste Kapillarmembransystem sowie das gegebenenfalls vorhandene Drainagesystem sowie gegebenenfalls vorhandene weitere Kapillarmembransysteme im Tascheninneren angeordnet sind.

14. System zur Wundbehandlung nach Anspruch 13, **dadurch gekennzeichnet, dass** die Unterseite der taschenförmigen Wundauflage aus einem vliesförmigen, flächigen Material oder einer semipermeablen, mikroporösen Flachmembran ausgebildet ist.

15. System zur Wundbehandlung nach einem oder mehreren der Ansprüche 1 bis 14, **dadurch gekennzeichnet, dass** erstes und zweites Kapillarmembransystem identisch sind und ein einziges Versorgungs-Kapillarmembransystem ausbilden, welches mit mindestens einer Versorgungsleitung verbunden ist, und dass der Filtermodul und der Entnahmebehälter über mindestens eine Versorgungsleitung des Versorgungs-Kapillarmembransystems mit dem Versorgungs-Kapillarmembransystem verbunden sind.

Fig. 1

Fig. 2

Fig. 3

Fig. 4

Europäisches
Patentamt

European
Patent Office

Office européen
des brevets

# EUROPÄISCHER RECHERCHENBERICHT

**Nummer der Anmeldung**

EP 15 16 7089

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | US 2005/107868 A1 (NAKAYAMA YASUHIDE [JP] ET AL) 19. Mai 2005 (2005-05-19) * Absätze [0021], [0022], [0053] - [0055], [0082] - [0084], [0124] * ----- | 1-15 | INV. A61F13/00 B01D61/02 A61K35/16 |
| Y | US 2013/046223 A1 (SCHRAMMEL STEPHEN [US]) 21. Februar 2013 (2013-02-21) * Ansprüche 1-21; Abbildungen 1-9 * ----- | 1-15 | |
| Y | DE 10 2010 030238 A1 (LMB LAB MED BLUTBANK TECHNOLOGIE GMBH [DE]) 22. Dezember 2011 (2011-12-22) * Ansprüche 1-12 * ----- | 1-15 | |
| Y | WO 2015/031654 A2 (CYTONICS CORP [US]) 5. März 2015 (2015-03-05) * Ansprüche 391-417; Abbildungen 1-19 * ----- | 1-15 | |

**RECHERCHIERTE SACHGEBIETE (IPC)**

A61F
B01D
A61K

Der vorliegende Recherchenbericht wurde für alle Patentansprüche erstellt

| Recherchenort | Abschlußdatum der Recherche | Prüfer |
|---|---|---|
| München | 1. Oktober 2015 | Gennari, Silvia |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument
 
& : Mitglied der gleichen Patentfamilie, übereinstimmendes
   Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 15 16 7089

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

01-10-2015

| Im Recherchenbericht angeführtes Patentdokument | Datum der Veröffentlichung | Mitglied(er) der Patentfamilie | Datum der Veröffentlichung |
|---|---|---|---|
| US 2005107868 A1 | 19-05-2005 | KEINE | |
| US 2013046223 A1 | 21-02-2013 | US 2013046223 A1<br>WO 2013025285 A1 | 21-02-2013<br>21-02-2013 |
| DE 102010030238 A1 | 22-12-2011 | DE 102010030238 A1<br>EP 2582415 A1<br>WO 2011157822 A1 | 22-12-2011<br>24-04-2013<br>22-12-2011 |
| WO 2015031654 A2 | 05-03-2015 | US 2015079194 A1<br>WO 2015031654 A2 | 19-03-2015<br>05-03-2015 |

EPO FORM P0461

Für nähere Einzelheiten zu diesem Anhang : siehe Amtsblatt des Europäischen Patentamts, Nr.12/82

**EP 3 092 987 A1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102006042732
- DE 29516471 U
- EP 0266683 A
- EP 1089778 A
- EP 1309363 A
- US 4639243 A
- WO 2011157822 A

- DE 3839567
- DE 4308850
- EP 0442147 A
- EP 1144096 A
- EP 0299381 A
- DE 2833493 A

**In der Beschreibung aufgeführte Nicht-Patentliteratur**

- HIMA-Vorschrift No. 3. Health Industry Manufacturers Association, 1982, vol. 4